# EUROPÄISCHE PATENTSCHRIFT

(11) **EP 1 536 755 B1**
(45) Veröffentlichungstag und Bekanntmachung des Hinweises auf die Patenterteilung: **22.11.2006**
(21) Anmeldenummer: 03788896.3
(22) Anmeldetag: 09.09.2003
(51) Int. Cl.: A61K 8/37, A61K 8/89, A61Q 1/10

(54) **WASSERBESTÄNDIGE MASCARA-ZUSAMMENSETZUNG MIT HOHEM WASSERGEHALT**
WATER-RESISTANT MASCARA COMPOSITION HAVING A HIGH WATER CONTENT
COMPOSITION DE MASCARA RESISTANT A L'EAU A FORTE TENEUR EN EAU

(30) Priorität: 12.09.2002 DE 10244117
(43) Veröffentlichungstag der Anmeldung: 08.06.2005
(73) Patentinhaber: Coty B.V., 2031 CC Haarlem (NL)
(72) Erfinder: MATEU, Juan, R., Oak Ridge, , NJ 07438 (US); CERNASOV, Domnica, Ringwood, NJ 07456 (US); MACCHIO, Ralph, Sparta, NJ 07971 (US)
(74) Vertreter: Walter, Wolf-Jürgen
(86) Internationale Anmeldenummer: PCT/EP2003/010038
(87) Internationale Veröffentlichungsnummer: WO 2004/032885

(56) Entgegenhaltungen:
- EP-A- 0 612 517
- FR-A- 2 707 162
- DATABASE WPI Section Ch, Week 200378 Derwent Publications Ltd., London, GB; Class A96, AN 2003-840601 XP002278804 & KR 2003 054 735 A (KOREAN COSMETICS CO LTD), 2. Juli 2003 (2003-07-02)

## Beschreibung

Die Erfindung betrifft eine wasserbeständige Mascara-Zusammensetzung, die zugleich einen hohen Wassergehalt hat.

Mascara-Zusammensetzungen mit guter Wasserbeständigkeit sind bereits bekannt. Häufig enthalten derartige Zusammensetzungen kosmetische Wachse.

Aus der US-A-5925337 ist eine wasserfeste Mascara-Zusammensetzung bekannt, die 2-40 Gew-% eines Wachses, 5-15 Gew-% eines Verdickungsmittels, 35-50 Gew-% eines flüchtigen organischen Lösungsmittels und 1-35 Gew-% eines wasserlöslichen filmbildenden Mittels enthält, wobei letzteres z.B. auch ein Acrylatpolymer sein kann. Die Zusammensetzung enthält keinen Emulgator. Der Wassergehalt dieser Formulierung liegt zwischen 7 und 12 Gew-%.

Der Erfindung liegt die Aufgabe zugrunde, eine Mascara-Zusammensetzung bereitzustellen, die einen sehr hohen Wassergehalt hat, hohen Glanz und gleichzeitig sehr gute Wasserbeständigkeiten.

Eine weitere Aufgabe besteht darin, eine Mascara zu entwickeln, die gut haftet aber auch leicht wieder mit Wasser von der Wimpernoberfläche entfernt werden kann.

Eine weitere Aufgabe besteht darin, solche Stoffe wie Talkum, Glimmer etc. ohne Phasentrennung in die Mascara-Zusammensetzung miteinzubeziehen.

Diese Aufgaben werden erfindungsgemäß gelöst durch eine Mascara-Zusammensetzung, die umfaßt
a) eine Ölphase, umfassend einen flüssigen Ester, ein Öl oder ein Gemisch davon, 1 bis 50 Gew-% eines Filmbildners auf Siliconbasis, 0,1 bis 10 Gew-% eines Gelbildners, ausgewählt unter Fettsäureestern, Glycolderivaten oder Gemischen davon;
b) 1 bis 50 Gew-% Substanzen, ausgewählt unter Pigmenten, Pulvern, Füllstoffen und Gemischen davon;
c) eine Wasserphase, umfassend 42 bis 75 Gew-% Wasser;
d) 0,1 bis 10 Gew-% eines oberflächenaktiven Mittels; und
e) weitere Trägerstoffe, Hilfsstoffe, Wirkstoffe oder Gemische davon bis 100 Gew-%,
wobei alle Prozentangaben auf das Gesamtgewicht der Zusammensetzung bezogen sind und wobei die Zusammensetzung frei von Wachsen und Kohlenwasserstofflösungsmitteln ist.

Da Wachsanteile immer Schmelztemperaturen bis 80 EC oder höher erfordern, ist es sowohl aus energetischen Gründen als auch bei Vorhandensein temperaturempfindlicher Bestandteile der Formulierung, wie Antioxidationsmittel, UV-Filter usw. vorteilhaft, die hohen Schmelztemperaturen zu vermeiden.

Der für die Ölphase eingesetzte Ester kann ein solcher sein wie Neopentyl Glycol Dioctanoate, Isopropyl Myristate, Diisopropyl Dimer Dilinoleate, Trimethylpropane Triisostearate, Triisostearyl Citrate, Cetearyl Octanoate, Distearyl Maleate etc., insbesondere Diisostearyl Maleate.

Besonders geeignete Öle sind beispielsweise Siliconöle, Mineralöle, Hydrogenated Polyisobuten, Polyisopren, Squalane, PPG-15-stearylether sowie pflanzliche Öle. Siliconöle wie beispielsweise Cycylomethicone oder Gemische verschiedener Siloxane, wie Dimethicone, sind bevorzugt. Bevorzugte Bereiche sind 0,1 bis 30 Gew-%.

Als Filmbildner auf Siliconbasis ist bevorzugt Trimethyl Siloxysilicate oder Amodimethicone oder ein Gemisch davon.

Ein bevorzugter Bereich für den Filmbildner ist 20 bis 30 Gew-%.

Normalerweise verringern hohe Anteile an derartigen Filmbildnern die viskosität in typischen Mascara-Zusammensetzungen. In der erfindungsgemäßen Zusammensetzung tritt diese Viskositätserniedrigung nicht auf, vermutlich wegen des Vorhandenseins des speziellen Gelbildners.

Wenn ein Fettsäureester als Gelbildner eingesetzt wird, sind Anteile davon im Bereich von 0,1 bis 5 Gew-% bevorzugt. Ein geeigneter Fettsäureester ist z.B. Stearyl Behenate, bevorzugt mit einem Anteil von 0,8-4 Gew-%.

Beispiele für Glycolderivate sind Glycerinbehenat, Glycerinstearat, Glycerinpalmitat, Glycerinarachidat. Ein bevorzugtes Glycolderivat ist Glycerol Behenate. Der Anteil dieser Glycolderivate liegt bevorzugt im Bereich von 1-4,5 Gew-%.

Ein besonders bevorzugter Gelbildner ist ein Gemisch von Glycolderivaten und Fettsäureestern, wie z.B. ein Gemisch aus Gylcerylbehenat und Stearylbehenat, vorteilhaft im Verhältnis 1:2 bis 4,0.

Die Wasserphase, die neben Wasser auch Hilfs- und weitere Wirkstoffe enthalten kann, umfaßt vorzugsweise 50 bis 75 Gew-% Wasser, bevorzugter 55 bis 75 Gew-% und speziell 57 bis 68 Gew-% Wasser.

Erfindungsgemäß werden als oberflächenaktive Mittel nichtionische oberflächenaktive Mittel bevorzugt, die im Bereich von 0,5 bis 7 Gew-% eingesetzt werden können. Dazu gehören Kokosnuß-acylmono- oder -diethanolamide, Alkylpolysaccharide, Lactobionamide, Ethylenglycolester, Glycerinmonoether, Polyhydroxyamide (Glucamide), primäre und sekundäre Alkoholethoxylate, insbesondere die C₈₋₂₀ aliphatischen Alkohole die durchschnittliche mit 1 bis 20 Molen Ethylenoxide pro Mol Alkohol ethoxyliert sind. Es können auch Gemische der zuvor genannten oberflächenaktiven Mittel eingesetzt werden. Bevorzugt ist Dimethicone Copolyol (Cetyl PEG/PPG-10/1 Dimethicone).

In der vorliegenden Erfindung einzusetzende Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische. Verbindungen wie gemahlene Festalgen, gemahlene Pflanzenteile, verkapselte und unverkapselte Getreidestärken sowie Glimmer-Titanoxid-organischer Farbstoff.

Der Anteil in der Zusammensetzung kann zwischen 0,1 und 50 Gew-% liegen, vorzugsweise 7-15 Gew-%.

Es können auch oberflächenbehandelte Pigmente enthalten sein, z.B. solche, die mit Alkylsilanen oder Perfluoralkoholphosphaten oberflächenbehandelt sind.

Zu verwendbaren kosmetischen Wirkstoffen gehören z. B. anorganische und organische Lichtschutzmittel, Radikalfänger, Feuchthaltemittel, Vitamine, Enzyme, pflanzliche Wirkstoffe, Polymere, Melanin, Antioxidationsmittel, entzündungswidrige natürliche Wirkstoffe, Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO 94/17588.

Die erfindungsgemäße Zusammensetzung kann vorteilhaft auch Antioxidationsmittel und Radikalfänger enthalten. Zu derartigen Substanzen gehören Vitamine wie Vitamin C und Derivate davon; beispielsweise Ascorbylacetate, -phosphate und - palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon;
α-Hydroxysäuren wie Citronensäure, Milchsäure, Apfelsäure; Stilbene und deren Derivate usw.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na-oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Als Hilfsstoffe können weiterhin Verdickungsmittel für die Wasserphase enthalten sein, wie z.B. Cellulosederivate, Hydrokolloide oder Salze von Polyacrylaten, wie beispielsweise Na-Polyacrylat.

Weitere Hilfsstoffe können feste Ester sein, wie z.B. solche mit 18 oder mehr Kohlenstoffatomen im Alkylteil. Dazu gehören bevorzugt Stearyl Behenate (Octadecylester von Docosansäure) und andere.

Als Feuchthaltemittel sind bevorzugt Glycerin, Butylenglycol, Propylenglycol oder Gemische davon mit Anteilen von 0,1 bis 20 Gew-%.

Ein Zusatz von Elektrolyten bewirkt eine Veränderung des Löslichkeitsverhaltens eines hydrophilen Emulgators. Hydrophile Emulgatoren unterliegen einer partiellen Phaseninversion, bei der eine Solubilisierung von Wasser durch die Ölphase auftritt. Dabei resultiert eine stabile Emulsion, insbesondere eine Mikroemulsion oder auch eine O/W/O-Emulsion. Geeignete Elektrolyte sind Salze mit den folgenden Anionen: Chloride, anorganische Oxo-Element-Anionen, wie Borate, Aluminate, Sulfate, Phosphate, Carbonate. Zu den auf organischen Anionen basierenden Elektrolyten gehören Citrate, Tartrate, Lactate, Propionate, Acetate und Benzoate sowie EDTA und deren Salze.

Kationen der Salze können sein Alkalimetall-, Erdalkalimetall-, Ammonium-, Alkylammonium-, Eisen-, Zink-Ionen.

Die Konzentrationen von Elektrolyten können im Bereich von 0,01-5 Gew-% liegen, vorzugsweise 0,1 bis 2,5 Gew-%.

Es wurde gefunden, daß übliche Wassergehalte in Mascaras, die normalerweise unter 20 Gew-% liegen, deutlich erhöht werden können, und zwar bis zu 75 Gew-% Wassergehalt. Weitere bevorzugte Bereiche sind 45-75 %, weiter bevorzugt 45-70 und speziell 50-75 % oder 55-70 Gew-%.

Darüber hinaus ist die erfindungsgemäße Mascara wasserfest, kann jedoch vollständig abgewaschen werden mit warmem Wasser bei einer Temperatur von etwa 29 EC und darüber.

Es ist weiterhin überraschend, daß solche problematischen Stoffe wie Talkum, Glimmer und synthetische Kügelchen trotz ihrer hydrophoben Eigenschaften und ihrer Neigung, die Wasserphase zu separieren, durch das Vorhandensein von Verdickungsmitteln, Gumme und Emulgatoren, wie z.B. ein kationisches Siliconpolymeres, wie Amodimethicone, im Bereich von 0,1 bis 20 Gew-%, vorzugsweise 0,1 bis 10 Gew-%, in eine stabile creme-artige Emulsion überführt werden kann. Auch Formulierungen ohne irgendwelche kationische Siliconpolymere sind möglich.

Es wurde weiterhin gefunden, daß trotz des Einsatzes von höheren Mengen an Siliconen für die Ölphase bzw. den Filmbildner (z.B. Cyclomethicone TMS) ein hoher Grad an Glanz für die Mascara-Zusammensetzung der Erfindung erreicht wurde, obwohl normalerweise derartige siliconhaltige Zusammensetzungen eher stumpf erscheinen. Dies ist ein weiterer bedeutender Vorteil gegenüber bekannten wasserfesten Mascara-Zusammensetzungen.

Da die erfindungsgemäße Mascara frei von Kohlenwasserstoff-Lösungsmitteln ist, ist keine spezielle Verpackung erforderlich. Es kann eine Verpackung wie für übliche Mascara auf Wasserbasis für die wasserfeste Mascara auf Wasserbasis verwendet werden, was sehr kosteneffektiv ist. Es sind keine Kosten für aufwendige neue und im höheren Maße abdichtende Verpackungen notwendig, die bei Mascaras auf Basis von Kohlenwasserstoff-Lösungsmitteln oder Ausrüstungen für die Verpackungen erforderlich wären.

Weiterhin zeigt die erfindungsgemäße Mascara nach einem klinischen Testbericht Wasserbeständigkeit wenigstens auf dem gleichen Niveau wie bei gängigen Marktprodukten. Sie zeigt darüber hinaus keinen signifikanten Anstieg an Streifenbildung (streaking), Verschmieren, Abblättern und/oder Verblassen nach drei Tauchvorgängen, festgestellt nach visueller Einschätzung unmittelbar danach und 6 Stunden später (Studie an 28 weiblichen Testpersonen mit üblichen Ein- und Ausschlußkriterien).

Die Erfindung soll nachstehend durch Beispiele näher erläutert werden. Alle Angaben erfolgen in Gewichtsprozent, sofern nichts anderes angegeben ist.

### Beispiel 1 Mascara I

| **Phase A** | |
|---|---|
| Purester 40 | 3,5 |
| Disteral Maleate | 0,5 |
| Glyceryl Behenate | 1,0 |
| (1:1) Cyclomethicone/Trimethyl | |
| Siloxysilcate (TMS) | 22 |
| Dimethicone Copolyol | 1, 1 |
| Schutzmittel | 0,8 |
| Propylene Glycol | 1,5 |
| Glycerine | 0,6 |

| **Phase B** | |
|---|---|
| Eisenoxid, schwarz | 7,8 |

| **Phase C** | |
|---|---|
| Wasser | 57 |
| Natriumchlorid | 0,2 |

| **Phase D** | |
|---|---|
| Dimethicone | 4 |

Phase A wird bei 200-1000 U/min vermischt und auf etwa 75°C bis zur Homogenität vermischt. Die Einführung von Glycerin und PPG zu Cyclomethicone/TMS führt zur Gelierung einer stabilen cremigen Paste. Phase B wird unter den gleichen Bedingungen wie Phase A eingebracht. Phase C wird bei 100-400 U/min gemischt, auf etwa 70°C erhitzt und in das Gemisch von A und B bei Aufrechterhaltung der Temperatur und mit 800-2000 U/min eingebracht. Das Gemisch wird unter Rühren auf 45-50°C abgekühlt. Danach wird die Phase D zugegeben, und das Gemisch wird auf 25-30°C abgekühlt.

### Beispiel 2-4 Mascara II, III und IV

| | **II** | **III** | **IV** |
|---|---|---|---|
| DISM (Diisostearyl Maleate) | 0,5 | 1 | 0,5 |
| (1:1) Cyclomethicone/TMS | 22 | 21 | 20 |
| Dimethicone Copolyol | 1,4 | 2 | 2 |
| Schutzmittel | 0,5 | 0,5 | 0,5 |
| Eisenoxide, schwarz | 6, 8 | 6,8 | 6,8 |
| Wasser | | q.s. ad 100 | |
| Purester 40* | 3 | 3,3 | 3 |
| Glyceryl Behenate | 1 | 2 | 1,2 |
| Mica 8 As | 1 | 1 | 1 |
| Amodimethicone | 1 | 0,8 | 0,8 |
| Merguard 1105 | 0,3 | 0,1 | 0,1 |
| Natriumchlorid | 0,4 | 0,6 | 0,6 |
| Butylene Glycol | 1,5 | 2,5 | 2,5 |
| Glycerine | 0,6 | 1 | 1 |
| Cyclomethicone | - | - | 2 |

| | | | |
|---|---|---|---|
| * Stearyl Behenate & Methyl Behenate & Stearyl Alcohol | | | |

Die Herstellung der Mascara war ähnlich wie im Beispiel 1.

### Beispiel 5 Vergleichsversuch Glanz

Es wurden Glanzmessungen unter Verwendung eines Glossgard System 60-Instruments (Gardner Instruments) bei einem Winkel von 85 Grad durchgeführt. Dieses Glanzmeter eignet sich besonders für die Messung von halbglänzenden Oberflächen wie Mascaras, Lippen- und Nägelprodukten. Es hat eine statistische Software mit einer Einheit für Mittelwertberechnungen und wiederholte Messungen.

Die Messungen erfolgten auf einem 6 mil (etwa 150 µm) dicken Basissubstrat, das 24 Stunden an Luft getrocknet wurde. Das Substrate war eine Basissubstratkarte Leneta Form 5c-Opazität.

Es wurden Vergleichsmessungen zwischen zwei wasserfesten marktüblichen Mascaras (A, B) und einer nicht-wasserfesten Glanzmascara (C) sowie der wasserfesten Mascara der vorliegenden Erfindung (D) durchgeführt.

**Ergebnisse bei einem Winkel von 85 Grad:**

| | | | |
|---|---|---|---|
| A | 2,7 | C | 27,8 |
| B | 0,6 | D | 79,6 |

Die Ergebnisse zeigen die Überlegenheit des Produktes der vorliegenden Erfindung auch hinsichtlich der Glanzeigenschaft.

## Patentansprüche

1. Wasserbeständige Mascara-Zusammensetzung, **dadurch gekennzeichnet, daß** die Zusammensetzung umfaßt
a) eine Ölphase, umfassend einen flüssigen Ester, ein Öl oder ein Gemisch davon, 1 bis 50 Gew-% eines Filmbildners auf Siliconbasis, 0,1 bis 10 Gew-% eines Gelbildners, ausgewählt unter Fettsäuren, Fettsäureestern, Glycolderivaten und Gemischen davon;
b) 1 bis 50 Gew-% Substanzen, ausgewählt unter Pigmenten, Pulvern, Füllstoffen und Gemischen davon;
c) eine Wasserphase, umfassend 42 bis 75 Gew-% Wasser;
d) 0,1 bis 10 Gew-% eines oberflächenaktiven Mittels; und
e) weitere Trägerstoffe, Hilfsstoffe, Wirkstoffe oder Gemische davon bis 100 Gew-%,
wobei die Zusammensetzung kein Wachs und kein Kohlenwasserstofflösungsmittel enthält und
wobei alle Prozentangaben auf das Gesamtgewicht der Zusammensetzung bezogen sind.

2. Zusammensetzung nach Anspruch 1, **dadurch gekennzeichnet, daß** das Öl ein Siliconöl ist.

3. Zusammensetzung nach Anspruch 1, **dadurch gekennzeichnet, daß** der Ester der Ölphase Diisostearyl Maleate ist.

4. Zusammensetzung nach Anspruch 1, **dadurch gekennzeichnet, daß** sie als Gelbildner ein Glycolderivat enthält, ausgewählt aus der Gruppe, bestehend aus Glycerinbehenat, Glycerinstearat, Glycerinpalmitat, Glycerinarachidat und Gemische davon.

5. Zusammensetzung nach Anspruch 4 **dadurch gekennzeichnet, daß** der Gelbildner im Bereich von 0,1 bis 5 Gew-% vorliegt, vorzugsweise 1-4,5 Gew-%.

6. Zusammensetzung nach Anspruch 1, **dadurch gekennzeichnet, daß** sie als Filmbildner Trimethyl Siloxysilicate enthält.

7. Zusammensetzung nach Anspruch 1, **dadurch gekennzeichnet, daß** die Wasserphase 50 bis 75 Gew-% Wasser umfaßt.

8. Zusammensetzung nach Anspruch 1, **dadurch gekennzeichnet, daß** die Wasserphase 56 bis 75 Gew-% Wasser umfaßt.

9. Zusammensetzung nach Anspruch 1, **dadurch gekennzeichnet, daß** das oberflächenaktive Mittel ein nichtionisches oberflächenaktive Mittel ist.

10. Zusammensetzung nach Anspruch 1, **dadurch gekennzeichnet, daß** sie Feuchthaltemittel enthält, ausgewählt unter Propylenglycol, Butylenglycol, Glycerin und Gemischen davon.

11. Mascara-Zusammensetzung nach Anspruch 1, umfassend eine Ölphase mit einem flüssigen Ester, 15 bis 30 Gew-% eines Filmbildners auf Siliconbasis und 0,1 bis 10 Gew-% eines Gelbildners auf Basis eines Fettsäureesters, eines Glycolderivates oder eiens Gemisches davon;
1 bis 15 Gew-% Pigmente, ausgewählt unter Eisenoxiden, Glimmer, Talkum, Kaolin, manganhaltigen Tonen, Nylonkügelchen, beschichteten Pigmenten und Gemischen davon;
eine Wasserphase, umfassend 45 bis 70 Gew-% Wasser;
0,5 bis 7 Gew-% eines nichtionischen oberflächenaktiven Mittels;
0,8 bis 2 Gew-% eines kationischen Silicon-Polymeren; und gegebenenfalls weitere Trägerstoffe, Hilfsstoffe, Wirkstoffe oder Gemische davon bis 100 Gew-%.
Zusammensetzung frei ist von Wachsen und Kohlenwasserstofflösungsmitteln.

## Claims

1. A water-proof mascara composition which comprises
a) an oil phase comprising a liquid ester, an oil or a mixture thereof, 1 to 50 % by weight of a film-forming agent on a silicone base, 0.1 to 10 % by weight of a gel-forming agent selected from among fatty acids, fatty acid esters, glycol derivatives and mixtures thereof;
b) 1 to 50 % by weight of substances selected from among pigments, powders, fillers and mixtures thereof;
c) a water phase comprising 42 to 75 % by weight of water;
d) 0.1 to 10 % by weight of a surface-active agent; and
e) ad 100 % by weight further carrier substances, auxiliaries, active agents or mixtures thereof,
all percentages being relative to the total weight of the composition, wherein the composition is free of waxes and hydrocarbon solvents.

2. Composition according to claim 1, wherein the oil of the oil phase is a silicone oil.

3. Composition according to claim 1, wherein the ester of the oil phase is Diisostearyl Maleate.

4. Composition according to claim 1, wherein the composition comprises as a gelling agent a glycol derivative selected from the group consisting of glyceryl behenate, glyceryl stearate, glyceryl palmitate, glyceryl arachidate and mixtures thereof.

5. Composition according to claim 1, wherein the gelling agent is in the range of 0.1-5 % by weight, preferably 1.0-4.5 % by weight.

6. Composition according to claim 1, wherein the composition comprises as a film forming agent Trimethyl Siloxysilicate.

7. Composition according to claim 1, wherein the water phase comprises 50 to 75 % by weight water.

8. Composition according to claim 7, wherein the water phase comprises 56 to 75 % by weight water.

9. Composition according to claim 1, wherein the surfactant is a nonionic surfactant.

10. Composition according to claim 1 wherein the said composition contains moisturizing substances selected from among Propylene Glycol, Butylene Glycol, Glycerine and mixtures thereof.

11. A mascara composition according to Claim 1 comprising an oil phase which comprises a liquid ester, 15 to 30 % by weight of a film-forming agent on a silicone base, and 0.1 to 10 % by weight of a fatty acid gel-forming agent on the basis of a or fatty acid ester, a glycol derivative or a mixture thereof;
1 to 15 % by weight of pigments selected from among iron oxides, mica, talc, kaolin, manganese containing clays, nylon pearls, coated pigments and mixtures thereof;
a water phase comprising 45 to 70 % by weight of water;
0.5 to 7 % by weight of a non-ionic surface-active agent;
0.8 to 2 % by weight of a cationic silicone polymer; and
optionally, ad 100 % by weight further carrier substances, auxiliaries, active agents or mixtures thereof, wherein the composition is free of waxes and hydrocarbon solvents.

## Revendications

1. Composition de mascara résistant à l'eau, **caractérisée en ce que** la composition comprend :
a) une phase huileuse comprenant un ester liquide, une huile ou un mélange de ceux-ci, 1 à 50 % en poids d'un filmogène à base de silicone, 0,1 à 10 % en poids d'un gélifiant choisi parmi les acides gras, les esters d'acides gras, les dérivés du glycol et des mélanges de ceux-ci ;
b) 1 à 50 % en poids de substances choisies parmi les pigments, les poudres, les charges et des mélanges de ceux-ci ;
c) une phase aqueuse comprenant 42 à 75 % en poids d'eau ;
d) 0,1 à 10 % en poids d'un tensioactif ; et
e) d'autres excipients, substances auxiliaires, substances actives ou des mélanges de ceux-ci jusqu'à obtenir 100 % en poids,
la composition ne contenant aucune cire et aucun solvant à base d'hydrocarbures et
tous les pourcentages étant exprimés par rapport au poids total de la composition.

2. Composition selon la revendication 1, **caractérisée en ce que** l'huile est une huile de silicone.

3. Composition selon la revendication 1, **caractérisée en ce que** l'ester de la phase huileuse est le maléate de diisostéaryle.

4. Composition selon la revendication 1, **caractérisée en ce qu'**elle contient un dérivé du glycol en tant que filmogène, choisi dans le groupe constitué par le béhénate de glycérol, le stéarate de glycérol, le palmitate de glycérol, l'arachidate de glycérol et des mélanges de ceux-ci.

5. Composition selon la revendication 4, **caractérisée en ce que** le filmogène se situe dans la plage de 0,1 à 5 % en poids, de préférence de 1 à 4,5 % en poids.

6. Composition selon la revendication 1, **caractérisée en ce qu'**elle contient du triméthyl siloxysilicate en tant que filmogène.

7. Composition selon la revendication 1, **caractérisée en ce que** la phase aqueuse comprend 50 à 75 % en poids.

8. Composition selon la revendication 1, **caractérisée en ce que** la phase aqueuse comprend 56 à 75 % en poids.

9. Composition selon la revendication 1, **caractérisée en ce que** le tensioactif est un tensioactif non ionique.

10. Composition selon la revendication 1, **caractérisée en ce qu'**elle contient un humectant choisi parmi le propylène glycol, le butylène glycol, le glycérol et des mélanges de ceux-ci.

11. Composition de mascara selon la revendication 1, comprenant une phase huileuse avec un ester liquide, 15 à 30 % en poids d'un filmogène à base de silicone et 0,1 à 10 % en poids d'un gélifiant à base d'un ester d'acide gras, d'un dérivé du glycol ou d'un mélange de ceux-ci ;
1 à 15 % en poids de pigments choisis parmi les oxydes de fer, le mica, le talc, le kaolin, les argiles manganésifères, les microbilles de nylon, les pigments enrobés et des mélanges de ceux-ci ;
une phase aqueuse comprenant 45 à 70 % en poids d'eau ; 0,5 à 7 % en poids d'un tensioactif non ionique ;
0,8 à 2 % en poids d'un polymère de silicone cationique et, le cas échéant, d'autres excipients, substances auxiliaires, substances actives ou mélanges de ceux-ci jusqu'à obtenir 100 % en poids. La composition est sans cires et sans solvants à base d'hydrocarbures.
